# EUROPÄISCHE PATENTSCHRIFT

(11) **EP 0 880 996 B1**
(45) Veröffentlichungstag und Bekanntmachung des Hinweises auf die Patenterteilung: **25.06.2003**
(21) Anmeldenummer: 98108422.1
(22) Anmeldetag: 08.05.1998
(51) Int. Cl.: B01J 25/00, C07C 209/48, C07B 31/00

(54) **Geformter Metall-Festbettkatalysator nach Raney, Verfahren zu seiner Herstellung und seine Verwendung**
Shaped fixed bed Raney-type metal catalyst,preparation process and use
Catalyseur métallique de Raney façonné utilisable en lit fixe, procédé de fabrication et utilisation

(30) Priorität: 26.05.1997 DE 19721897
(43) Veröffentlichungstag der Anmeldung: 02.12.1998
(73) Patentinhaber: Degussa AG, 40474 Düsseldorf (DE)
(72) Erfinder: Sauer, Jörg, Dr., 63517 Rodenbach (DE); Haas, Thomas, Dr., 60316 Frankfurt/Main (DE); Keller, Bruno, Dr., 55263 Wackernheim (DE); Freund, Andreas, Dr., 63801 Kleinostheim (DE); Burkhardt, Werner, 63636 Brachttal (DE); Michelchen, Dietrich, 63526 Erlensee (DE); Berweiler, Monika, 63477 Maintal (DE)

(56) Entgegenhaltungen:
- EP-A- 0 648 534
- EP-A- 0 771 784
- EP-A- 0 842 699
- DE-A- 4 446 907
- CURRY-HYDE H E ET AL: "IMPROVEMENTS TO RANEY COPPER METHANOL SYNTHESIS CATALYSTS THROUGH ZINC IMPREGNATION. PORE STRUCTURE AND THE INFLUENCE ON ACTIVITY" APPLIED CATALYSIS A: GENERAL, Bd. 95, 1993, Seiten 65-74, XP002065792

## Beschreibung

Die Erfindung betrifft einen geformten Metall-Festbettkatalysator nach Raney, welcher wenigstens eine Katalysatorlegierung aus einem Katalysatormetall und einer auslaugbaren Legierungskomponente enthält, wobei der Katalysator in einer Schale mit einer Dicke von 0,1 bis 2,0 mm ausgehend von der Oberfläche des geformten Katalysators durch vollständiges oder teilweises Auslaugen der auslaugbaren Legierungskomponente aktiviert ist und gegebenenfalls Promotoren enthält. Ebenso betrifft die Erfindung ein Verfahren zur Herstellung des Katalysators durch Mischen eines Pulvers aus der Katalysatorlegierung mit einem hochmolekularen Polymer, Verformen der Mischung zu Formkörpern und Entfernen des Polymers durch Temperaturbehandlung sowie Kalzinieren der Formkörper bei Temperaturen unter 850 °C. Ein weiterer Gegenstand der Erfindung betrifft die Verwendung des Katalysators für Hydrierungen, Dehydrierungen und Hydrogenolysen.
Aktivierte Metallkatalysatoren sind in der chemischen Technik als Raney-Katalysatoren bekannt. Sie werden überwiegend in Pulverform bei einer Großzahl von Hydrierreaktionen an organischen Verbindungen eingesetzt.

Diese pulverigen Katalysatoren werden aus einer Legierung eines katalytisch aktiven Metalls, im folgenden auch als Katalysatormetall bezeichnet, mit einer in Alkalien löslichen weiteren Legierungskomponente hergestellt. Als Katalysatormetalle kommen hauptsächlich Nickel, Kobalt, Kupfer oder Eisen zum Einsatz. Für die in Alkalien lösliche Legierungskomponente wird vorwiegend Aluminium eingesetzt, aber auch andere Komponenten sind verwendbar, insbesondere sind auch Zink und Silizium geeignet.

Diese sogenannte Raney-Legierung wird gemäß dem Verfahren nach Raney zunächst fein vermahlen. Anschließend wird das Aluminium durch Auslaugen mit Alkalien, wie zum Beispiel Natronlauge, ganz oder teilweise entfernt.
Damit wird das Legierungspulver aktiviert. Durch Auslaugen des Aluminiums weist das Legierungspulver eine hohe spezifische Oberfläche zwischen 20 und 100 m²/g auf und ist reich an adsorbiertem Wasserstoff. Das aktivierte Katalysatorpulver ist pyrophor und wird unter Wasser, organischen Lösungsmitteln oder durch Einbetten in hochsiedende organische Verbindungen gelagert.

Pulverkatalysatoren haben den Nachteil, dass sie nur in Batch-Verfahren eingesetzt werden können und nach der katalytischen Umsetzung durch aufwendige Filtration von den Reaktionsmedien abgetrennt werden müssen. Es sind daher verschiedene Verfahren zur Herstellung von Formkörpern bekannt geworden, die nach Auslaugen des Aluminiums zu aktivierten Metall-Festbettkatalysatoren führen.

Das US-Patent 4,826,799 beschreibt die Herstellung von aktivierten Metall-Festbettkatalysatoren nach Raney durch Vermischen eines Pulvers der Legierung aus Katalysatormetall und Aluminium mit einem organischen Polymer und gegebenenfalls einem Verformungshilfsmittel, Verformen dieser Mischung durch Extrusion oder Verpressen zu den gewünschten Formkörpern und Kalzinieren der Formkörper an Luft bei Temperaturen über 850 °C. Dies führt durch Verbrennen der organischen Beimengungen zu einer Porenstruktur der Formkörper und zur Bildung von α-Aluminiumoxid, welches als keramisches Bindemittel zwischen den Legierungspartikeln wirkt und den Formkörpern die gewünschte mechanische Stabilität verleiht. Daran schließt sich die Aktivierung der Formkörper durch Auslaugen des restlichen, während des Kalzinierens nicht oxidierten, Aluminiums an.

Entscheidendes Merkmal dieses Verfahrens ist die Bildung von α-Aluminiumoxid zwischen den Legierungspartikeln als keramischer Binder. α-Aluminiumoxid ist im Gegensatz zu γ-Aluminiumoxid und Aluminium nicht in Alkalien löslich und wird deshalb beim Aktivieren des Formkörpers mit Natronlauge nicht herausgelöst.

Die gemäß US 4,826,799 hergestellten Katalysatoren weisen gravierende Nachteile auf. Zur Bildung von α-Aluminiumoxid müssen die Formkörper oberhalb von 850 °C kalziniert werden. Unterhalb von 850 °C bildet sich nämlich kein α-Aluminiumoxid, sondern nur das in Alkalien lösliche γ-Aluminiumoxid. Das als Bindemittel verwendete α-Aluminiumoxid ist katalytisch inaktiv und mindert somit die Katalsatoraktivität. Während des Kalzinierens bildet sich auf der Oberfläche der Legierungspartikel eine mehr oder weniger geschlossene Schicht aus diesem inaktiven, in Alkalien unlöslichen, Material. Dadurch wird die Aktivierung der Legierung erschwert. Im fertigen Katalysator stellt diese Schicht eine Diffusionsbarriere für die Eduktmoleküle dar, was weitere Aktivitätseinbußen zur Folge hat.

Darüber hinaus wird von modernen Katalysatorsystemen zum Schutz der Umwelt eine leichte Wiederaufarbeitbarkeit gefordert. Die Aufarbeitung der keramisch gebundenen Metall-Festbettkatalysatoren ist jedoch wegen des unlöslichen keramischen Bindemittels schwierig. Die EP 0 648 534 A1 beschreibt die Herstellung eines aktivierten Metall-Festbettkatalysators, welcher ohne α-Aluminiumoxid als Bindemittel auskommt. Der Katalysator wird erhalten durch Verformen eines Pulvers wenigstens einer Katalysatorlegierung mit einem Pulver der reinen Katalysatormetalle unter Zusatz von Verformungshilfsstoffen und Porenbildnern und anschließendem Kalzinieren bei Temperaturen unter 850 °C. Beim Kalzinieren verbrennen die Verformungshilfsstoffe und Porenbildner. Legierungspulver und Metallpulver sintern dabei zu mechanisch stabilen und porösen Formkörpern zusammen. Diese Formkörper bestehen somit aus den Partikeln der Katalysatorlegierungen, die durch Pulver der reinen Katalysatormetalle gebunden sind. Sei weisen keine katalytisch inaktiven, keramischen Bindemittel auf. Durch Auslaugen des in den Katalysatorlegierungen enthaltenen Aluminiums mit Natronlauge werden die Formkörper in einer oberflächlichen Schale aktiviert.

Obwohl das bei diesem Katalysator als Bindemittel verwendete reine Katalysatormetall auch einen gewissen Beitrag zur katalytischen Aktivität des Katalysators leistet, ist sein Beitrag jedoch wegen der geringen spezifischen Oberfläche dieses Materials vernachlässigbar. Somit ist die auf die Katalysatormasse bezogenene katalytische Aktivität des Katalysators geringer als sie bei Verzicht auf das Katalysatormetall als Bindemittel sein könnte.

Die EP 0 648 534 A1 empfiehlt zur Erhöhung der Festigkeit der Katalysatorformkörper, als Bindemittel Pulver der reinen Katalysatormetalle zu verwenden, deren Korngröße kleiner ist als die Korngröße der Legierungspulver. Dies führt zu relativ dichten Katalysatoren mit geringem Porenvolumen. Die EP 0 648 534 A1 nennt keine Schüttdichten für die Katalysatoren. Die nach dieser Schrift hergestellten Festbettkatalysatoren weisen jedoch sehr hohe Schüttdichten von etwa 2 Kg/l auf.

Aus der DE 40 07 345 A1 sind thermoplastische Massen für die Herstellung metallischer Formkörper bekannt. Die Massen enthalten A) ein sinterbares pulverförmiges Metall oder eine pulverförmige Metallegierung, B) eine Mischung aus B1) einem Polyoxymethylenhomo- oder copolymerisats und B2) einem in B1) homogen gelösten oder mit einer mittleren Teilchengröße von weniger als 1 µm in B1) dispergierten Polymer als Bindemittel sowie ein Dispergierhilfsmittel. Diese Massen können zu Formkörpern verformt werden. Zur Entfernung des Bindemittels werden die nach der Verformung erhaltenen Grünkörper mit einer gasförmigen, säurehaltigen Atmosphäre behandelt. Die Behandlung wird so lange durchgeführt, bis der Polyoxymethylenanteil zu mindestens 80 % entfernt ist. Anschließend wird das so erhaltenen Produkt auf 250 bis 500 °C erhitzt, um den noch vorhandenen Rest des Bindemittels vollständig zu entfernen. Das vom Bindemittel befreite Produkt kann durch Sintern in einen metallischen Formkörper Überführt werden, der auch bei großen Wandstärken frei von Rissen und Poren ist.

Die DE-A 44 46 907 beschreibt in Beispiel 1 einen Nickelkatalysator, der nach einer Calcinierung bei 900 °C mit 20%iger Natronlauge bei 90 °C ausgelaugt wird. An keiner Stelle dieses Beispiels 1 beziehungsweise des Dokumentes wird ausgeführt, wie dick die Schale ist, die aktiviert wurde. Weiterhin wird an keiner Stelle ausgeführt, welches Gesamtporenvolumen der Katalysator aufweist. Auch findet sich an keiner Stelle des Dokumentes ein Hinweis auf das Schüttgewicht des Katalysators.

Die EP-A-0 842 699 beschreibt einen Festbettkatalysator nach Raney. Den Beispielen 1, 2 und 3 kann entnommen werden, dass der Grünling, welcher aus einer Kobaltaluminiumlegierung hergestellt wird, bei der Temperatur von 900 °C calciniert wird. Der Katalysator gemäß dem Dokument weist somit eine Aluminiumoxidmodifikation auf, die bei der anschließenden Behandlung mit Natronlauge nicht aus dem Katalysator herausgelöst werden kann, und ist somit nicht bindemittelfrei.

Aufgabe der vorliegenden Erfindung ist es, einen geformten Metall-Festbettkatalysator anzugeben, welcher bei gleicher oder besserer Hydrieraktivität eine wesentlich geringere Schüttdichte als vergleichbare Katalysatoren aus dem Stand der Technik aufweist.

Diese Aufgabe wird durch einen geformten Metall-Festbettkatalysator, enthaltend wenigstens eine Katalysatorlegierung aus einem oder mehreren Katalysatormetallen aus der Reihe Nickel, Kobalt, Kupfer und/oder Eisen und einer auslaugbaren Legierungskomponente und gegebenenfalls Promotoren,
dadurch gekennzeichnet,
dass die bei der Katalysatorenherstellung eingesetzten Körner der Katalysatorenlegierung eine mittlere Korngröße im Bereich zwischen 30 und 120 µm, der Katalysator ausschließlich aus der oder den Katalysatorlegierungen besteht, frei von alpha- und gamma-Aluminiumoxid ist, ein Gesamtporenvolumen von 0,1 bis 0,6 ml/g und eine Schüttdichte von kleiner 2,2 kg/l aufweist, und in einer Schale mit einer Dicke von 0,1 bis 2,0 mm, ausgehend von der Oberfläche, durch vollständiges oder teilweises Auslaugen der äuslaugbaren Legierungskomponente aktiviert ist, gelöst.

Der Katalysator enthält also im Vergleich zu den Katalysatoren nach der EP 0 648 534 A1 kein minderaktives reines Katalysatormetall als Binder. Darüber hinaus weist er bei Verwendung gleicher Legierungspulver ein höheres Porenvolumen als der bekannte Katalysator auf, was bei Anwendung gleicher Aktivierungsbedingungen zu einer aktivierten Schale mit größerer Dicke führt. Diese Unterschiede ergeben eine höhere spezifische Aktivität des erfindungsgemäßen Katalysators sowohl bezogen auf seine Masse als auch auf seine Schüttdichte.

Als Katalysatormetalle werden Nickel, Kobalt, Kupfer oder Eisen und als auslaugbare Legierungskomponente Aluminium, Zink oder Silizium eingesetzt. Das Gewichtsverhältnis zwischen Katalysatormetall und der auslaugbaren Legierungskomponente in der Katalysatorlegierung liegt, wie bei Raney-Legierungen üblich, im Bereich zwischen 30 : 70 und 70 : 30.

Die erfindungsgemäßen Katalysatoren können zur Beeinflussung ihrer katalytischen Aktivität noch mit anderen Metallen dotiert sein. Ziel einer solchen Dotierung ist zum Beispiel die Verbesserung der Selektivität in einer bestimmten Reaktion. Die Dotierungsmetalle werden häufig als Promotoren bezeichnet. Dotierung bzw. Promotierung von Raney-Katalysatoren werden zum Beispiel in der USP 4,153,578, in der DE-AS 21 01 856, in der DE-OS 21 00 373 und in der DE-AS 20 53 799 beschrieben. Geeignete Promotoren sind Chrom, Eisen, Kobalt, Tantal, Titan und/oder Molybdän sowie auch die Metalle der Platingruppe. Zweckmäßigerweise werden sie schon als Legierungsbestandteil der Katalysatorlegierung zugefügt. Typischerweise beträgt ihr Anteil an der Katalysatorlegierung bis zu 15 Gew.-%. Im Falle der Dotierung mit Molybdän ist es zweckmäßig, die Dotierung erst nach dem Aktivieren des Katalysators vorzunehmen. Dazu wird der fertige Katalysator in eine Molybdatlösung von erhöhter Temperatur, z. B. bei 80 °C, eingebracht. Abhängig von der Länge der Behandlungsdauer wird eine bestimmte Menge der Molybdänverbindung vom Katalysator adsorbiert.

Die Herstellung eines aktivierten Metall-Festbettkatalysators gemäß Erfindung erfolgt durch Mischen eines Legierungspulvers aus einem katalytisch aktivem Katalysatormetall, gegebenenfalls Promotoren und einer auslaugbaren Legierungskomponente mit einem hochmolekularen Polymer, Verformen der Mischung zu Grünkörpern und Entfernen des Polymers durch Temperaturbehandlung sowie Kalzinieren der Grünkörper bei Temperaturen unter 850 °C und Aktivieren der erhaltenen Formkörper durch Auslaugen der auslaugbaren Legierungskomponente mittels Natronlauge. Als hochmolekulares Polymer wird eine Polyoxymethylenhomo- oder copolymer-Formmasse verwendet, welche durch Temperaturbehandlung bei Temperaturen zwischen 100 und 300 °C zersetzt wird. Im folgenden wird statt Polyoxymethylenhomo- oder Polyoxymethylencopolymer-Formmasse der Ausdruck Polyoxymethylen (POM) verwendet. Polyoxymethylen und Verfahren zu seiner Herstellung sind dem Fachmann bekannt und in der Literatur beschrieben.

Das Polyoxymethylen dient als Bindemittel für die Grünkörper sowie als Porenbildner. Es wird direkt mit der Katalysatorlegierung vermischt. Dabei hat es sich gezeigt, dass bei Verwendung von Polyoxymethylen mit einem Volumen-Fließindex MVR (nach DIN ISO 1133, gemessen bei 190 °C und einer Belastung von 2,16 Kg) zwischen 1 und 50, bevorzugt im Bereich von 5 bis 13, besonders bevorzugt im Bereich von 6 bis 9, auf weitere Zusätze zur Mischung weitgehend verzichtet werden kann. Der MVR Fließindex stellt eine für die Zwecke der vorliegenden Erfindung ausreichende Charakterisierung von Polyoxymethylen dar.

Die Verwendung von POM mit einem MVR von kleiner 1 hat sich auf Grund der durch das Vermischen mit der Katalysatorlegierung reduzierten Schmelzviskosität als ungünstig erwiesen. Der Einsatz von POM mit einem MVR von größer 50 scheitert aus Gründen der schlechten Eigenschaften als Bindemittel für die Mischung.

Katalysatorlegierung und Polymer werden in Form von Pulvern bei 180 bis 250 °C zu einer verformbaren Masse verknetet. Dabei werden die Primär-Kornspektren der eingesetzten Legierungspulver im Wesendichen nicht verändert. Es findet also keine Vermahlung statt. Ziel dieser Vorbehandlung ist die Vorbereitung der Mischung für das nachfolgende Formgebungsverfahren. Anwendbar sind beispielsweise die Extrusion, Tablettierung und Kompaktierung. Bevorzugt wird die Mischung zu Strangpresslingen mit Durchmessern von etwa 1 bis 8 mm extrudiert, die in etwa 2 bis 5 mm lange Stücke zerteilt werden. Im Falle der Extrusion werden Legierungspulver und Polymer dem Extruder separat zugeführt. Die Vermischung beider Komponenten erfolgt im Extruder.

Die mittlere Korngröße der verwendeten Katalysatorlegierung liegt im Bereich zwischen 30 und 120 µm. Kleinere Komdurchmesser als 30 µm führen zu Formkörpern mit einer für die Katalysatoranwendung zu geringen Porosität. Bei Korndurchmessern über 120 µm wird die Porosität zu groß und die Festigkeit der Formkörper lässt nach. Das Polyoxymethylen wird der Mischung bevorzugt in einer Menge von 5 bis 100 Gew.-% bezogen auf die Menge der Katalysatorlegierung zugesetzt.

Die durch die Formgebung erhaltenen Grünkörper werden einer Temperaturbehandlung unterzogen, um das Polyoxymethylen zu im Wesentlichen Formaldehyd zu zersetzen und aus dem Grünkörper auszutreiben. Die Zersetzung von Polyoxymethylen beginnt bei Temperaturen oberhalb von etwa 100 °C. Um ein Zerplatzen der Grünkörper durch zu starke Ausgasung der Zersetzungsprodukte zu vermeiden, sollte die Erwärmung der Formkörper entsprechend langsam vorgenommen werden. Bewährt haben sich Zersetzungsraten von etwa 6 bis 10 g Zersetzungsprodukte pro Kilogramm eingesetztem Polyoxymethylen. Diese Zersetzungsrate kann durch entsprechende Anpassung der Temperatur eingestellt werden. Bei gleichbleibender Temperatur sinkt die Zersetzungsrate mit fortschreitender Zersetzung ab. Zur Beschleunigung der vollständigen Zersetzung empfiehlt es sich daher die Temperatur während der Zersetzung kontinuierlich anzuheben, um die Zersetzungsrate während des gesamten Zersetzungsprozesses in etwa konstant zu halten. Der Zersetzungsprozess ist dann je nach gewählter Zersetzungsrate nach 170 bis 200 Minuten abgeschlossen. Erfahrungsgemäß muss dabei die Temperatur der Grünkörper von 100 °C bis auf etwa 300 °C erhöht werden.

Die Grünkörper können also zunächst relativ schnell bis auf etwa 100 °C erwärmt werden. Daran schließt sich eine geregelte Temperaturerhöhung bis 300 °C an, die eine langsame Zersetzung des Polyoxymethylens gewährleistet. Wird der Grünkörper zu schnell auf 300 °C erhitzt kommt es zu einer schlagartigen Zersetzung des Polyoxymethylens und damit zu einer Zerstörung der Formkörper. Nach Abschluss der Zersetzung kann die Temperatur der Grünkörper in etwa 100 bis 140 Minuten auf die Kalzinierungstemperatur von bevorzugt 800 °C erhöht werden. Die Grünkörper werden dann bei dieser Temperatur für die Dauer von 60 bis 180 Minuten kalziniert.

Die zweckmäßige Temperaturrampe für die Zersetzung des Polyoxymethylens kann vom Fachmann in wenigen orientierenden Versuchen ermittelt werden. Hierbei ist zu berücksichtigen, dass durch die Temperaturrampe auch die Porosität des fertigen Katalysators in gewissen Grenzen beeinflusst werden kann. Durch die Zersetzung des Polyoxymethylens wird nämlich der Grünkörper etwas aufgebläht. Wie schon oben ausgeführt wurde, darf dieser Vorgang allerdings nicht zu einer völligen Zerstörung des Grünkörpers führen. Er kann jedoch gezielt ausgenutzt werden, um die Porosität des fertigen Katalysatorformkörpers einzustellen. Die Zersetzung des Polyoxymethylens kann unter Luft erfolgen. Zur Unterstützung der Zersetzung kann sie jedoch auch, wie in der DE 40 07 345 A1 beschrieben, in einer säurehaltigen Atmosphäre vorgenommen werden. Geeignete Säuren für die Behandlung sind anorganische oder organische Säuren, die bei den angewendeten Temperaturen verdampfbar sind. Geeignete Säuren sind zum Beispiel Salpetersäure, Ameisensäure oder Essigsäure.

Auch die Kalzinierung der Grünkörper kann an Luft vorgenommen werden. Die Begrenzung der Kalzinierungstemperatur auf Werte unterhalb von 850 °C gewährleistet, dass das dabei eventuell gebildet Aluminiumoxid nur in Form von γ-Aluminiumoxid vorliegt, welches bei der nachfolgenden Aktivierung aus den Formkörpern herausgelöst wird.

Zur Aktivierung werden die Formkörper nach Abkühlung in einer 20 Gew.-% tigen Lauge, bevorzugt Natronlauge, bei einer Temperatur von 80 °C und für die Dauer von 120 Minuten behandelt. Dabei wird die in der Katalysatorlegierung enthaltene auslaugbare Legierungskomponente, gewöhnlich Aluminium, aus der Katalysatorlegierung herausgelaugt. Die Auslaugung schreitet von der Oberfläche des Formkörpers aus in die Tiefe fort. Bei den angegebenen Werten für die Konzentration der Natronlauge, ihre Temperatur sowie die Dauer der Behandlung werden bei einem Porenvolumen der Formkörper von 0,3 ml/g aktivierte Schalendicken von etwa 0,8 mm Dicke erhalten. Durch Ändern der genannten Parameter kann die Schalendicke in gewissen Grenzen variiert werden. Die angegebenen Auslaugparameter sind also keine starren Größen, sondern können vom Fachmann seinen Erfordernissen angepasst werden. Nach der Auslaugung werden die Formkörper mit Wasser alkalifrei gewaschen und unter Wasser bis zur Verwendung aufbewahrt.

Das beschriebene Verfahren ermöglicht die Herstellung eines aktivierten Metall-Festbettkatalysators, der vollständig aus der Katalysatorlegierung besteht. Gegenüber dem in der EP 0 648 534 A1 beschriebenen Katalysator benötigt er also überraschenderweise kein reines Katalysatormetall als Bindemittel und weist daher eine höhere volumenspezifische Aktivität auf. Es hat sich auch gezeigt, dass mit dem Verfahren prinzipiell Katalysatoren mit geringeren Schüttgewichten als gemäß der EP 0 648 534 A1 zugänglich sind. Dies ist bei teuren Katalysatormetallen wie Kobalt besonders vorteilhaft.

Der erfindungsgemäße Katalysator lässt sich für die Hydrierung, Dehydrierung und Hyrogenolyse organischer und anorganischer Subtrate verwenden. Unter Verwendung des erfindungsgemäßen Katalysators lassen sich beispielsweise Nitroverbindungen, Imine, Nitrile, CC-Doppel- und C-C-Dreifachbindungen, aromatische und heteroaromatische Ringe, Carbonylverbindungen und Epoxide, ferner CO und CO₂ unter für derartige Hydrierungen üblichen Bedingungen mit Wasserstoff hydrieren. Ferner lassen sich beispielsweise Alkohole zu Carbonsäuren und Aminoalkanole zu Aminocarbonsäuren dehydrieren.

Eine besonders bevorzugte Verwendung richtet sich auf Verfahren zur Herstellung von Isophorondiamin (IPDA) aus Isophoronnitril, wobei in einer ersten Stufe in an sich bekannter Weise in Gegenwart eines sauren Iminierungskatalysators Isophoronnitril mit Ammoniak in das entsprechende Iminnitril überführt und dieses in einer zweiten Stufe in Gegenwart des erfindungsgemäßen Katalysators zu Isophorondiamin aminierend hydriert wird. Die erste Stufe wird in An- oder Abwesenheit eines Lösungsmittels, vorzugsweise in Gegenwart eines niederen Alkohols, bei 0 bis 100 °C durchgeführt, beispielsweise gemäß DE-Patentanmeldung 196 27 265.3 in Gegenwart eines Sulfonatgruppen enthaltenden Organopolysiloxans als Iminierungskatalysator. Bei der zweiten Stufe wird das Reaktionsgemisch der Iminierungsstufe vorzugsweise unter Rieselbettfahrweise bei einem Druck von 3 bis 10 Mpa über den erfindungsgemäßen Katalysator geleitet, wobei die Reaktionstemperatur entweder 80 bis 150 °C oder zunächst 10 bis 90 °C und anschließend über 90 bis 150 °C beträgt - bezüglich weiterer Details der Verfahrensführung wird der Offenbarungsinhalt der DE 43 43 890 A1 und DE 43 43 891 hier eingeschlossen.

### Beispiel 1:

Es wurde ein aktivierter Kobaltkatalysator unter Verwendung einer Kobalt/Aluminiumlegierung mit 50 Gew.-% Aluminium bezogen auf das Gesamtgewicht der Legierung nach dem beschriebenen Verfahren hergestellt. Die mittlere Korngröße der Kobaltlegierung betrug 60 µm.

In einem Doppelwellenextruder (Werner & Pfleiderer; Extruder ZSK 30) wurde eine bei Raumtemperatur hergestellte Mischung, bestehend aus 15 Gew.-% eines Polyoxymethylencopolymeren und 85 Gew.-% der Kobalt/Aluminiumlegierung bei einer Temperatur von 190 °C mit einem Massenstrom von 10 Kg/h extrudiert. Das Polyoxymethylencopolymere enthielt 2,7 Gew.-% Butandiolformal als Comonomeres (Ultraform® N2320) und wies einen MVR (190°C, 2,16 Kg) von 6,7 - 8,5 auf.

Zur Zersetzung des Polyoxymethylens wurden die Grünkörper in einem Ofen zunächst innerhalb von 10 Minuten auf 120 °C erwärmt. Die Zersetzung erfolgte dann bei kontinuierlicher Temperatursteigerung von 120 auf 280 °C innerhalb von 90 Minuten. Nach dieser Zeit war die Zersetzung weitgehend abgeschlossen. Danach wurde die Temperatur innerhalb von 125 Minuten auf 800 °C gesteigert. Bei dieser Temperatur wurden die Grünkörper für weitere 140 Minuten kalziniert.

Nach dem Erkalten der Formkörper wurden sie in Natronlauge (20 Gew.-%) bei einer Temperatur von 80 °C für die Dauer von 120 Minuten aktiviert.

Die fertigen Katalysatorformkörper hatten einen Durchmesser von 5 mm, eine Länge von ebenfalls 5 mm und besaßen eine aktivierte Schalendicke von 0,8 mm. Ihre Bruchfestigkeit betrug 120 N (gemessen in radialer Richtung nach ASTM D 4179-82). Der erfindungsgemäß hergestellte Katalysator zeichnete sich durch eine gegenüber dem Stand der Technik erheblich verringerte Schüttdichte von nur 1,2 Kg/l aus und erreichte trotzdem noch eine für katalytische Anwendungen ausreichende Festigkeit.

### Vergleichsbeispiel 1:

Es wurde ein Vergleichskatalysator gemäß EP 0 648 534 A1 hergestellt. Hierzu wurde das Legierungspulver von Beispiel 1, ein Kobaltpulver mit einer mittleren Korngröße von 20 µm sowie als Gleitmittel und Porenbildner ein Wachspulver (Ethylenbisstearoylamid) mit einer mittleren Korngröße von 15 µm verwendet.

Legierungspulver und 15 Gew.% Kobaltpulver, bezogen auf das Legierungspulver, wurden unter Zugabe von Wasser in einem Mischer sorgfältig homogenisiert und nach einer Zwischentrocknung mit 2,5 Gew.-% Wachspulver, bezogen auf das Legierungspulver, vermischt. Die so erhaltene Masse wurde zu Tabletten mit einem Durchmesser von 5 mm und einer Höhe von ebenfalls 5 mm verpresst. Die Tabletten wurden anschließend wie in Beispiel 1 kalziniert und aktiviert. Die fertigen Katalysatorkörper wiesen eine aktive Schalendicke von 0,3 mm und eine Schüttdichte von 2,2 Kg/l auf.

### Anwendungsbeispiel:

Der nach Beispiel 1 hergestellte Katalysator (K 1), der Vergleichskatalysator nach Vergleichsbeispiel 1 (VK 1)und ein handelsüblicher Kobalt-Trägerkatalysator (Kobalt auf silikatischem Träger) (VK 2) wurden bei der Herstellung von 3-Aminomethyl-3,5,5-trimethycyclohexylamin (Isophorondiamin, IPDA) aus 3-Cyano-3,5,5-trimethylcyclohexanon (Isophoronnitril, IPN) in einem zweistufigen Verfahren auf ihre katalytische Wirksamkeit geprüft. Das Verfahren ist im wesentlichen in der DE 195 40 191 C1 beschrieben. Die Eigenschaften der verwendeten Katalysatoren sind in Tabelle 1 aufgelistet.

In der ersten Stufe wurde dabei Isophoronnitril in Anwesenheit eines Iminierungskatalysators mit Ammoniak und unter Zugabe von Methanol zumindest teilweise in 3-Cyano-3,5,5-trimethylcyclohexanimin überführt und in der zweiten Stufe an einem Kobalt-Festbettkatalysator aus Tabelle 1 bei einer Temperatur von 100 °C und einem Druck von 6 MPa mit Wasserstoff aminierend hydriert.

Jede Stufe der Herstellung von IPDA wurde in Abweichung von der in der DE 195 40 191 C1 beschriebenen Vorgehensweise in separaten Reaktoren durchgeführt. Beide Reaktoren waren jedoch hintereinandergeschaltet. Sie wurden durch getrennte Ölheizungen temperiert.

Das erste Reaktorrohr hatte einen Innendurchmesser von 20 mm und eine Länge von 250 mm und war mit 30 ml eines Sulfonatgruppen enthaltenden Organopolysiloxans (Korngröße 0,4 bis 1,4 mm; Schüttdichte 525 g/l) als Iminierungskatalysator befüllt (siehe DE-Patentanmeldung 196 27 265.3).

Der Hydrierreaktor besaß einen Innendurchmesser von 17 mm und eine Länge von 350 mm und wurde bei jedem Versuch mit 150 ml des jeweils zu prüfenden Katalysators befüllt.

Die Temperatur des ersten Reaktors wurde auf 35 °C und die Temperatur im zweiten Reaktor auf 100 °C eingestellt. Der Druck in beiden Reaktoren betrug 6 MPa.

Die Einsatzlösung aus IPN (15 Gew.-%), Ammoniak (30 Gew.-%) und Methanol (55 Gew.-%) wurde mit einem Massenstrom von 80 ml/h von unten durch das erste Reaktionsrohr gepumpt; das dabei erhaltene iminierte Reaktionsgemisch lief von dort auf den zweiten Reaktor. Der Wasserstoff wurde von oben in das zweite Reaktionsrohr mit einem Volumenstrom von 36 l/h eingeleitet, der Reaktor also als Rieselbettreaktor betrieben. Die Produktflüssigkeit wurde unter dem zweiten Reaktor in einem Abscheidegefäß aufgefangen. Das aufgefangene Produktgemisch wurde auf IPDA und entsprechende Nebenprodukte gaschromatografisch untersucht. Die Untersuchungsergebnisse sind in Tabelle 2 aufgeführt.

**Tabelle 1:**

| Eigenschaften der Katalysatoren | | | |
|---|---|---|---|
| | K1 | VK1 | VK2 |
| Abmessungen | 5 Ø x 5 | 5 Ø x 5 | 4,5 Ø x 5 |
| Kobalt [Gew.-%] | 72 | 81 | 45 |
| Aluminium [Gew.-%) | 28 | 19 | n. b. |
| Schüttdichte [Kg/l] | 1,2 | 2,2 | 0,74 |
| Porenvolumen [cm³/g] | 0,3 | 0,05 | 0,3 |
| Schalendicke [mm] | 0,8 | 0,3 | n. b. |
| Bruchfestigkeit [N] | 120 | 300 | 80 |
| n. b.: nicht bestimmt | | | |

**Tabelle 2:**

| Ergebnisse der IPDA-Herstellung | | | |
|---|---|---|---|
| | K1 | VK1 | VK2 |
| IPDA-Ausbeute | 89,7 | 89,1 | 84,3 |
| Produktreinheit (% IPDA) | 99,9 | 99,75 | 99,85 |

Aus den in Tabelle 2 aufgelisteten Ergebnissen lässt sich ableiten, dass mit dem erfindungsgemäßen Katalysator bei gleichem Katalysatorvolumen eine leicht erhöhte Zielproduktausbeute erreicht wird. Da gleichzeitig unerwünschte Nebenprodukte in geringerem Umfang gebildet werden, wird nach Reindestillation eine deutlich verbesserte Reinheit erhalten. Auf Grund seiner geringeren Schüttdichte verringern sich die Rohstoffkosten des Katalysators gegenüber dem nach EP 0 648 534 A1 hergestellten Katalysator VK1 erheblich.

## Patentansprüche

1. Geformter Metall-Festbettkatalysator, enthaltend wenigstens eine Katalysatorlegierung aus einem oder mehreren Katalysatormetallen aus der Reihe Nickel, Kobalt, Kupfer und/oder Eisen und einer auslaugbaren Legierungskomponente und gegebenenfalls Promotoren,
**dadurch gekennzeichnet,**
**dass** die bei der Katalysatorenherstellung eingesetzten Körner der Katalysatorenlegierung eine mittlere Korngröße im Bereich zwischen 30 und 120 µm, der Katalysator ausschließlich aus der oder den Katalysatorlegierungen besteht, frei von alpha- und gamma-Aluminiumoxid ist, ein Gesamtporenvolumen von 0,1 bis 0,6 ml/g und eine Schüttdichte von kleiner 2,2 kg/l aufweist, und in einer Schale mit einer Dicke von 0,1 bis 2,0 mm, ausgehend von der Oberfläche, durch vollständiges oder teilweises Auslaugen der auslaugbaren Legierungskomponente aktiviert ist.

2. Geformter Katalysator nach Anspruch 1,
**gekennzeichnet durch** Aluminium, Zink oder Silizium als auslaugbare Legierungskomponente und einem Gewichtsverhältnis zwischen den Katalysatormetallen und der auslaugbaren Legierungskomponente von 30 : 70 bis 70 : 30.

3. Geformter Katalysator nach Anspruch 2,
**dadurch gekennzeichnet,**
**dass** er noch mit bis zu 15 Gew.-%, bezogen auf das Gewicht der Katalysatorlegierungen mit den Elementen Chrom, Eisen, Kobalt, Tantal, Molybdän und/oder Titan als Promotoren dotiert ist.

4. Verfahren zur Herstellung eines aktivierten Metall-Festbettkatalysators nach einem der vorstehenden Ansprüche durch Mischen wenigstens eines Legierungspulvers aus einem Katalysatormetall, gegebenenfalls Promotoren und einer auslaugbaren Legierungskomponente mit einem hochmolekularen Polymer, Verformen der Mischung zu Grünkörpern und Entfernen des Polymers durch Temperaturbehandlung sowie Kalzinieren der Grünkörper bei Temperaturen unter 850 °C und Aktivieren der erhaltenen Formkörper durch Auslaugen der auslaugbaren Legierungskomponente mittels einer Lauge,
**dadurch gekennzeichnet,**
**dass** als hochmolekulares Polymer ein Polyoxymethylenhomo- oder copolymer mit einem Volumen-Fließindex MVR (nach DIN ISO 1133, gemessen bei 190 °C und einer Belastung von 2,16 kg) zwischen 1 und 50, bevorzugt im Bereich von 5 bis 13, besonders bevorzugt im Bereich von 6 bis 9, verwendet und welches durch Temperaturbehandlung bei Temperaturen zwischen 100 und 300 °C zersetzt wird.

5. Verfahren nach Anspruch 4,
**dadurch gekennzeichnet,**
**dass** die Katalysatorlegierung eine mittlere Korngröße von 30 bis 120 µm und das Polyoxymethylen in einer Menge von 5 bis 100 Gew.-% bezogen auf die Menge Katalysatorlegierung der Mischung zugesetzt wird.

6. Verfahren nach Anspruch 5,
**dadurch gekennzeichnet,**
**dass** das hochmolekulare Polymer unter Einwirkung eines sauren Mediums bei Temperaturen zwischen 100 und 300 °C zersetzt wird, wobei durch Regelung der Aufheizrate und/oder durch Dosierung des sauren Mediums eine in etwa konstante Zersetzungsrate von 6 bis 10 g Formaldehyd pro Kilogramm eingesetztem Polyoxymethylen und Minute eingestellt wird.

7. Verwendung des geformten Metall-Festbettkatalysators gemäß einem der Ansprüche 1 bis 3 oder erhalten gemäß einem der Ansprüche 4 bis 6 zur Durchführung von Hydrierungen, aminierenden Hydrierungen, Dehydrierungen und Hydrogenolysen.

## Claims

1. A shaped metal fixed-bed catalyst comprising at least one catalyst alloy of one or more catalyst metals selected from the group consisting of nickel, cobalt, copper and iron and a leachable alloying component and, if desired, promoters, **characterized in that** the particles of catalyst alloy used in the production of the catalyst have a mean particle size in the range from 30 to 120 µm, the catalyst consists exclusively of the catalyst alloy(s), is free of alpha- and gamma-aluminium oxide, has a total pore volume of from 0.1 to 0.6 ml/g and has a bulk density of less than 2.2 kg/l, and has been activated in an outer zone having a thickness of from 0.1 to 2.0 mm, starting from the surface, by complete or partial leaching of the leachable alloying component.

2. A shaped catalyst according to claim 1, **characterized by** aluminium, zinc or silicon as leachable alloying component and a weight ratio of catalyst metals to the leachable alloying component of from 30:70 to 70:30.

3. A shaped catalyst according to claim 2, **characterized in that** it is additionally doped with up to 15% by weight, based on the weight of the catalyst alloys, with the elements chromium, iron, cobalt, tantalum, molybdenum and/or titanium as promoters.

4. A process for producing an activated metal fixed-bed catalyst according to any one of the preceding claims by mixing at least one alloy powder comprising a catalyst metal, if desired promoters and a leachable alloying component with a high molecular weight polymer, shaping the mixture to form green bodies and removing the polymer by heat treatment and calcining the green bodies at temperatures below 850°C and activating the shaped bodies obtained by leaching out the leachable alloying component by means of an alkali, **characterized in that** a polyoxymethylene homopolymer or copolymer having a volume flow index MVR (in accordance with DIN ISO 1133, measured at 190°C and a load of 2.16 kg) of from 1 to 50, preferably in the range from 5 to 13, particularly preferably in the range from 6 to 9, is used as high molecular weight polymer and this is decomposed by heat treatment at temperatures of from 100 to 300°C.

5. A process according to claim 4, **characterized in that** the catalyst alloy has a mean particle size of from 30 to 120 µm and the polyoxymethylene is added in an amount of from 5 to 100% by weight, based on the amount of catalyst alloy in the mixture.

6. A process according to claim 5, **characterized in that** the high molecular weight polymer is decomposed by action of an acidic medium at temperatures of from 100 to 300°C, and an approximately constant decomposition rate of from 6 to 10 g of formaldehyde per kilogram of polyoxymethylene used per minute is set by regulating the heating rate and/or by metering of the acidic medium.

7. The use of the shaped metal fixed-bed catalyst according to any one of claims 1 to 3 or obtained according to any one of claims 4 to 6 for carrying out hydrogenations, aminative hydrogenations, dehydrogenations and hydrogenolyses.

## Revendications

1. Catalyseur en lit fixe métallique moulé, contenant au moins un alliage de catalyseur à base d'un ou plusieurs métaux de catalyseur choisis dans la série du nickel, du cobalt, du cuivre et/ou du fer, et d'un composant d'alliage lessivable et éventuellement de promoteurs,
**caractérisé en ce que**
les grains d'alliage de catalyseur mis en oeuvre pour la production du catalyseur possèdent une taille moyenne de grains dans la plage entre 30 et 120 µm, le catalyseur consiste exclusivement en le ou les alliage(s) de catalyseur, est dépourvu d'oxyde d'aluminium alpha, et gamma, possède un volume total de pores de 0,1 à 0,6 ml/g et une densité apparente inférieure à 2,2 kg/l, et est activé dans une coquille ayant une épaisseur de 0,1 à 2 mm en partant de la surface, par un lessivage complet ou partiel du composant d'alliage lessivable.

2. Catalyseur moulé selon la revendication 1,
**caractérisé par**
la présente d'aluminium, de zinc ou de silicium, comme composant d'alliage lessivable et par un rapport en poids entre les métaux du catalyseur et les composants d'alliage lessivables de 30 :70 à 70 :30.

3. Catalyseur moulé selon la revendication 2,
**caractérisé en ce qu'**
il est dopé encore jusqu'à 15 % en poids rapporté au poids des alliages de catalyseur avec les éléments chrome, fer, cobalt, tantale, molybdène, et/ou titane comme promoteurs.

4. Procédé de production d'un catalyseur en lit fixe métallique activé selon l'une des revendications précédentes par mélange d'un alliage en poudre à base d'un métal de catalyseur, éventuellement de promoteurs et d'un composant d'alliage lessivable avec un polymère de haut poids moléculaire, mise en forme du mélange en composés verts, et élimination du polymère par traitement thermique ainsi que calcination des solides verts à des températures en dessous de 850°C et activation des solides moulés obtenus par lessivage des composants d'alliage lessivables à l'aide d'une lessive,
**caractérisé en ce que**
comme polymère à haut poids moléculaire on utilise un polyoxyméthylène homo- ou copolymère ayant un indice d'écoulement en volume MVR (selon la norme DIN ISO 1133, mesuré à 190°C et à une charge de 2,16 kg) entre 1 et 50, de préférence dans la zone de 5 à 13 et d'une manière particulièrement préférée de 6 à 9 et on le décompose à des températures comprises entre 100 et 300°C.

5. Procédé selon la revendication 4,
**caractérisé en ce que**
l'alliage de catalyseur possède une taille moyenne de grains de 30 à 120 µm et que le polyoxyméthylène est ajouté en une quantité de 5 à 100 % en poids rapporté à la quantité d'alliage du catalyseur, au mélange.

6. Procédé selon la revendication 5,
**caractérisé en ce que**
le polymère de haut poids moléculaire est décomposé par action d'un milieu acide à des températures comprises entre 100 et 300°C, pour lequel on ajuste par réglage de la vitesse d'échauffement et/ou par admission dosée du milieu acide, une vitesse de décomposition à peu près constante de 6 à 10 g de formaldéhyde par kilogramme de polyoxyméthylène mis en oeuvre et par minute.

7. Utilisation du catalyseur en lit fixe métallique conformément à l'une des revendications 1 à 3 ou bien obtenu conformément à l'une des revendications 4 à 6, en vue de l'exécution d'hydrogénations, d'hydrogénations aminantes, de déshydrogénations et d'hydrogénolyses.
